(19) Europäisches Patentamt
European Patent Office
Office européen des brevets

(11) Veröffentlichungsnummer: **0 538 704 B1**

(12) **EUROPÄISCHE PATENTSCHRIFT**

(45) Veröffentlichungstag der Patentschrift: **12.10.94**

(51) Int. Cl.[5]: **C07C 37/16**, C07C 37/18, C07C 39/367

(21) Anmeldenummer: **92117393.6**

(22) Anmeldetag: **12.10.92**

# (54) Verfahren zur Monobenzylierung von p-substituierten Phenolen.

(30) Priorität: **24.10.91 DE 4135073**
**28.11.91 DE 4139053**

(43) Veröffentlichungstag der Anmeldung:
**28.04.93 Patentblatt 93/17**

(45) Bekanntmachung des Hinweises auf die Patenterteilung:
**12.10.94 Patentblatt 94/41**

(84) Benannte Vertragsstaaten:
**CH DE FR GB IT LI**

(56) Entgegenhaltungen:
**EP-A- 0 003 338**
**EP-A- 0 278 211**
**US-A- 1 880 566**

**CHEMICAL ABSTRACTS, vol. 89, no. 1, 3. Juli 1978, Columbus, Ohio, US; abstract no. 6109b, Z. VODAK ET AL. '2-Benzyl-4-chlorophenol.' Seite 521 ;Spalte 1 ;**

**CHEMICAL ABSTRACTS, vol. 79, no. 3, 23. Juli 1973, Columbus, Ohio, US; abstract no. 18265f, A.R. ABDURASULEVA ET AL. 'Alkylation of phenols and their ethers in the presence of small amounts of protic catalysts. Benzylation and cyclohexylation of phenol and anisole.' Seite 416 ;Spalte 1 ;**

**CHEMICAL ABSTRACTS, vol. 91, no. 1, 2. Juli 1979, Columbus, Ohio, US; abstract no. 5000p, N. ISMAILOV ET AL. 'Benzylation of cresols.' Seite 469 ;Spalte 1 ;**

(73) Patentinhaber: **BAYER AG**

**D-51368 Leverkusen (DE)**

(72) Erfinder: **Langer, Reinhard, Dr.**
**Scheiblerstrasse 111**
**W-4150 Krefeld (DE)**
Erfinder: **Buysch, Hans-Josef, Dr.**
**Brandenburger Strasse 28**
**W-4150 Krefeld (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die Erfindung betrifft ein Verfahren zur Benzylierung von p-substituierten Phenolen in o-Position.

Es ist bekannt, daß bei der Benzylierung von Phenolen stets Gemische aus 2-Benzyl-, 4-Benzyl- und 2,6-Dibenzylphenolen sowie höhersiedenden Kondensationsprodukten in unterschiedlicher Zusammensetzung erhalten werden.

So ist z.B. aus J. Amer. Chem. Soc. 53, 2379 (1931) bekannt, daß bei der Kondensation von p-Kresol mit Benzylalkohol in Gegenwart von Aluminiumchlorid ein Produktgemisch entsteht, welches je nach dem Mengenverhältnis der Ausgangsstoffe 30 bis 36 Gew.-% an dibenzylierten Folge- und Nebenreaktionsprodukten enthält.

Weiterhin ist bekannt [Abdurasuleva et. al., Zh. Org. Khim. 9, 132 (1973], daß bei der Benzylierung von 4-Chlorphenolen mit Benzylchlorid in Gegenwart von Katalysatoren wie $FeCl_3$, $FeSO_4$ oder $ZnSO_4$ bis zu 18 Mol% Dibenzylchlorphenole entstehen. Darüber hinaus wird in der Tschechischen Patentschrift 170 972 [Chemical Abstracts 89, (1978), 6109 b] die Kondensation von 4-Chlorphenol mit Benzylchlorid im Molverhältnis 4:1 in Gegenwart von 10 Gew.-% eines sulfonierten Styrol-Divinylbenzol-Copolymerisats (stark saurer Kationentauscher) zu 2-Benzyl-4-chlorphenol beschrieben. Die Ausbeute an dem gewünschten Produkt beträgt zwar 83 %, bezogen auf umgesetztes 4-Chlorphenol; es entsteht jedoch auch eine größere Menge an höhersiedenden Folge-und Nebenreaktionsprodukten; das Verhältnis von gebildetem 2-Benzyl-4-chlorphenol zu den höhersiedenden Produkten beträgt 4,16:1.

Neben der geringen Selektivität und damit geringen Eduktausnutzung benötigen die beschriebenen Verfahren einen großen Phenolüberschuß und lange Reaktionszeiten, bedingt durch einen langsamen Eintrag des Benzylierungsmittels und eine Nachreaktionsperiode (z.B. in CS 170 972 3+2=5 Stunden), wodurch eine geringe Raumzeitausbeute erzielt wird. Ferner muß eine große Menge an Katalysator abgetrennt und regeneriert oder vernichtet werden.

In EP 278 211 werden Zeolithe des Faujasit-Typs als Katalysatoren für die Umsetzung von p-substituierten Phenolen mit gegebenenfalls substituierten Benzylierungsmitteln beschrieben. Mit 10 Gew.-% Na-Y als Katalysator kann bei 200 °C binnen 3 Stunden 1 Mol Benzylalkohol mit 4 Molen 4-Chlorphenol quantitativ umgesetzt werden, wobei das Verhältnis von gebildetem 2-Benzyl-4-Chlorphenol zu höhersiedenden Produkten mit 9,9:1 deutlich besser als in CS 170 972 ist. Die Abtrennung und gegebenenfalls Regenerierung des eingesetzten Katalysators ist jedoch technisch umständlich.

Es wurde nun gefunden, daß man die gewünschte Monobenzylierung p-substituierter Phenole in der o-Position mit hoher Raumzeitausbeute und hoher Selektivität in einem vollkontinuierlichen Prozess erreichen kann, wenn man die p-substituierten Phenole mit gegebenenfalls substituierten Benzylhalogeniden, Benzylestern, Benzylalkoholen oder Benzylethern oder Gemischen aus diesen Halogeniden, Estern, Alkoholen und Ethern in einer Destillationsapparatur in Gegenwart mindestens eines löslichen sauren Katalysators umsetzt.

Die Erfindung betrifft daher ein Verfahren zur Herstellung von in p-Stellung substituierten o-Benzylphenolen der Formel

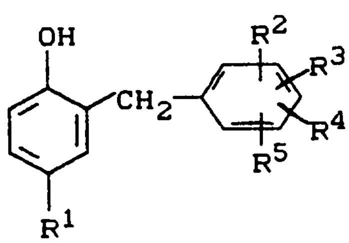

(I)

in der

$R^1$ für Halogen, Hydroxy, $C_1$-$C_{12}$-Alkyl, $C_1$-$C_{12}$-Alkoxy, $C_1$-$C_{12}$-Alkylmercapto, $C_6$-$C_{12}$-Aryl, $C_6$-$C_{12}$-Aryloxy oder $C_6$-$C_{12}$-Arylmercapto steht und

$R^2$ bis $R^5$ unabhängig voneinander Wasserstoff, Halogen, die Cyanogruppe, $C_1$-$C_{12}$-Alkyl, $C_1$-$C_{12}$-Alkoxy, $C_1$-$C_{12}$-Alkylmercapto, $C_6$-$C_{12}$-Aryl, $C_6$-$C_{12}$-Aryloxy oder $C_6$-$C_{12}$-Arylmercapto bedeuten und zwei der Reste $R^2$ bis $R^5$, wenn sie benachbart sind, Trimethylen, Tetramethylen oder den Rest eines ankondensierten Benzolringes bedeuten,

durch Umsetzung von p-substituierten Phenolen der Formel

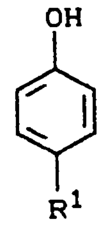

(II),

in der

$R^1$ die oben genannte Bedeutung besitzt,

mit Benzylverbindungen der Formel

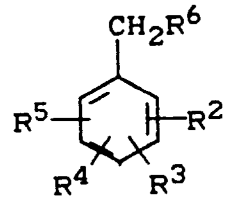

(III),

in der

$R^2$ bis $R^5$ die oben genannte Bedeutung besitzen und

$R^6$ für Halogen, $C_1$-$C_{12}$-Alkylcarboxy, substituiertes oder nichtsubstituiertes Phenylcarboxy, $C_1$-$C_{12}$-Alkyl- oder Phenylsulfatoxy, Hydroxy, $C_1$-$C_{12}$-Alkoxy, substituiertes oder nichtsubstituiertes Benzyloxy oder substituiertes oder nichtsubstituiertes p-$R^1$-Phenoxy, Trichloracetyloxy oder Methansulfonyloxy steht, wobei $R^1$ die oben genannte Bedeutung besitzt,

das dadurch gekennzeichnet ist, daß die Umsetzung in einer kontinuierlich arbeitenden Destillationsapparatur in Gegenwart mindestens eines sauren, löslichen Katalysators in einer Menge zwischen 10 und 0,001 Mol-%, bezogen auf Äquivalente an zudosierter Benzylverbindung, durchgeführt wird, wobei in einem Druckbereich zwischen 1 mbar und 10 bar und in einem Temperaturbereich zwischen 100 und 240 °C im Reaktionsteil der Kolonne und in einem Temperaturbereich oberhalb desjenigen im Reaktionsteil, aber unterhalb von 350 °C im unteren Teil der Kolonne gearbeitet wird.

Der Reaktionsablauf läßt sich wie folgt darstellen:

(II) + (III) → (I) + H-$R^6$ (Spaltprodukt)

Als Alkylreste der oben genannten Formeln, auch in den Alkoxy-Substituenten, kommen solche mit 1 bis 12, bevorzugt 1 bis 4, Kohlenstoffatomen in Frage, die geradkettig oder verzweigt sein können. Beispielsweise werden genannt: Methyl, Ethyl, n-Propyl, iso-Propyl, n-Butyl, iso-Butyl, sec.-Butyl, tert.-Butyl, Pentyl, Hexyl, Octyl, Decyl, Dodecyl, bevorzugt Methyl, Ethyl, n-Propyl, iso-Propyl, n-Butyl, iso-Butyl, sec.-Butyl sowie tert.-Butyl, besonders bevorzugt Methyl.

Als Halogen sei genannt: Fluor, Chlor, Brom und Jod, bevorzugt Fluor, Chlor und Brom, besonders bevorzugt Chlor und Brom, ganz besonders bevorzugt Chlor.

Alkyl, Alkoxy, Phenyl und Benzyl können ein- oder zweifach durch Methyl, Ethyl, Methoxy, Ethoxy oder Chlor substituiert sein.

Bevorzugte p-substituierte Phenole für das erfindungsgemäße Verfahren sind solche der Formel

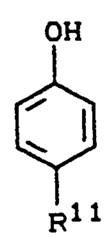

(IV),

in der

$R^{11}$ für Fluor, Chlor, Brom, Hydroxy, $C_1$-$C_4$-Alkyl, $C_1$-$C_4$-Alkoxy, Phenyl oder Phenoxy steht.

Besonders bevorzugte p-substituierte Phenole für das erfindungsgemäße Verfahren sind solche der Formel

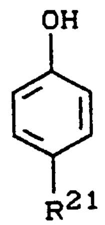

(V),

in der

$R^{21}$ für Chlor, Brom, Hydroxy, Methyl oder Methoxy steht.

Bevorzugte Benzylverbindungen der Formel (III) sind solche, in denen $R^5$ für Wasserstoff steht. Besonders bevorzugte Benzylverbindungen der Formel (III) sind solche, in denen $R^4$ und $R^5$ für Wasserstoff stehen.

Für das erfindungsgemäße Verfahren können z.B. folgende p-substituierte Phenole eingesetzt werden: p-Chlorphenol, p-Kresol, Hydrochinon-monoethylether, p-Bromphenol, p-Hydroxybiphenyl, 4-Chlor-4'-hydroxybiphenyl sowie p-Fluorphenol, bevorzugt p-Chlorphenol, p-Kresol und Hydrochinon-monoethylether, besonders bevorzugt p-Chlorphenol und p-Kresol.

Als Benzylierungsmittel kommen beispielsweise in Frage: Benzylchlorid, Benzylbromid, Benzylacetat, Benzylformiat, Benzyltrichloracetat, Benzylvaleriat, Dibenzylsulfat, Benzylmesylat (Methansulfonsäure-benzylester), Benzylalkohol, Dibenzylether, p-Chlorphenyl-benzylether, p-Methylbenzylalkohol, Bis-p-methylbenzylether, p-Hydroxymethyl-chlorbenzol, Bis-p-chlor-benzylether, bevorzugt Benzylchlorid, Benzylalkohol, die Dibenzylether und p-Chlorphenyl-benzylether.

Erfindungsgemäß werden die Phenole und die Äquivalente an Benzylierungsmittel in einem Molverhältnis größer als 2, bevorzugt größer als 4, besonders bevorzugt größer als 8, ganz besonders bevorzugt größer als 12 umgesetzt, die Obergrenze der Molverhältnisse beträgt 50, bevorzugt 40, besonders bevorzugt 30. Dieser Phenolüberschuß tritt im erfindungsgemäßen Verfahren jedoch ausschließlich innerhalb des kontinuierlich arbeitenden Reaktors auf und spiegelt sich in der erzielten Selektivität wieder.

Es stellt einen besonderen Vorteil bei dem erfindungsgemäßen Verfahren dar, daß die Edukte annähernd equimolar in den Reaktor dosiert werden und die Produkte rein, beziehungsweise vorgereinigt dem Reaktor an getrennten Stellen entnommen werden, die Umsetzung jedoch bei einem gewünschten und gezielt einstellbaren mehr oder weniger großen Phenolüberschuß vonstatten geht.

Das erfindungsgemäße Verfahren wird in einer kontinuierlich arbeitenden Destillationsapparatur durchgeführt, in der Druck und Reaktionstemperatur insoweit miteinander verknüpft sind, als durch den angelegten Druck die Siedetemperaturen und damit das Temperaturprofil in der Destillationskolonne bestimmt werden.

Die Apparatur zur Durchführung des erfindungsgemäßen Verfahrens besteht im einfachsten Fall aus einer mit Füllkörpern oder mit einer Destillationspackung bestückten isolierten Kolonne zur Durchführung einer kontinuierlichen Destillation, wie sie Stand der Technik ist.

Eine solche Apparatur besitzt i.a. einen Wärmetauscher am Fuß der Kolonne und einen Kondensator mit Rückflußteiler am Kolonnenkopf. Am Fuß der Kolonne wird das Produkt gegebenenfalls zusammen mit di- und höherbenzylierten Nebenprodukten ausgeschleust. Eine mögliche Verfahrensweise besteht darin, das Produkt (I) frei von Hochsiedern in einem Seitenstrom aus dem unteren Teil der Kolonne zu entnehmen und so rein zu isolieren; am Fuß der Kolonne werden dann die produktfreien Hochsieder entnommen.

Die Kolonne wird vorteilhaft oberhalb der Ausschleusezone für das Produkt mit einem zusätzlichen Wärmetauscher versehen.

Dieser Wärmetauscher wird mit dem gesamten oder einem Teilstrom der herabfließenden abreagierten Reaktionsmischung beschickt und dient dazu, den größten Teil des in der Kolonne "eingeschlossenen" Überschußphenols (II) zu verdampfen, um den unteren Destillationsbereich zur Reindestillation oder Teilreinigung des Produktes zu entlasten.

Die Kolonne wird ferner vorteilhaft unterhalb des Kolonnenkopfes mit einem weiteren zusätzlichen Wärmetauscher versehen. Dieser dient dazu, den größten Teil des Phenolgehaltes der anströmenden Gasphase niederzuschlagen, bevorzugt mit Energierückgewinnung, z.B. durch Dampferzeugung, um den

oberen Teil der Kolonne zur Abtrennung des leichtsiedenden Reaktionsproduktes von Überschußphenol zu entlasten. Die Kolonne kann daher, mit oder ohne zusätzliche Wärmetauscher, in drei Zonen eingeteilt werden, in die obere Zone zur Abtrennung des leichtsiedenden Spaltproduktes $H\text{-}R^6$ vom Phenolüberschuß, in den unteren Teil zur Abtrennung des Produktes vom Überschußphenol (II) und gegebenenfalls von hochsiedenden Nebenprodukten und in den mittleren Teil, in dem (dem Destillationsvorgang überlagert) die Reaktion (II + III → I + $H\text{-}R^6$) abläuft. Alle drei Zonen werden entsprechend ihrer Flüssigkeit- und Gasbelastung gegebenenfalls mit unterschiedlichen Kolonnenpackungen und Kolonnendurchmessern versehen. Gegebenenfalls kann eine Destillationsapparatur in obigem Sinne auch aus mehreren mit Rohrleitungen für die Brüden und Flüssigkeitsströme verbundenen hintereinander geschalteten Destillationskolonnen bestehen.

Die zu verwendenden Füllkörper beziehungsweise geordneten Packungen sind die für Destillationen an sich üblichen, wie sie beispielsweise in Ullmanns Encyclopädie der Techn. Chemie Bd. 2, S. 528 ff (4. Auflage) oder in den Firmenschriften z.B. von Sulzer, Montz, Raschig, Kühni oder Norton beschrieben sind.

Genannt seien beispielsweise:
Raschig-® oder Pallringe®, Berl-Intalex- oder Torussättel®, Interpackkorper aus verschiedenen Materialien, wie Glas, Steinzeug, Porzellan, Kohlenstoff, Edelstahl, Kunststoff, die (bes. als Metall) gewebe- oder maschenartig verarbeitet sein können.

Bevorzugt sind Füllkörper und geordnete Packungen, die eine große Oberfläche und gute Benetzung sowie eine ausreichende Verweilzeit der Flüssigkeit aufweisen, beispielsweise Pall-® und Novolax-Ringe®, Berlsättel, BX-Packungen®, Montz-Pak®, Mellapak®, Melladur®, Kerapak® und CY-Packungen®.

Für das erfindungsgemäße Verfahren geeignet sind jedoch nicht nur Füllkörperkolonnen, sondern auch solche mit festen Einbauten. Geeignet sind Bodenkolonnen mit Sieb-, Glocken-, Ventil-, Tunnel- und Zentrifugalböden, die wiederum in unterschiedlichen Ausführungen vorliegen können. Bevorzugt sind z.B. Glocken- und Ventilböden mit hohen Verweilzeiten bei gutem Stoffaustausch.

In der Ausführung mit zwei zusätzlichen Wärmetauschern, die den Reaktionsteil der Kolonne einrahmen, erhält der Reaktionsteil bevorzugt einen größeren Druckmesser als die anderen Kolonnenabschnitte.

Die Querschnittsfläche des Reaktionsteils ist bevorzugt mehr als doppelt, besonders bevorzugt mehr als dreimal, ganz besonders bevorzugt mehr als viermal so groß wie die des unteren Kolonnenabschnittes.

Die drei Komponenten: Phenol (II), Benzylierungsmittel (III) und Katalysator können getrennt gegebenenfalls an unterschiedlichen Stellen der Kolonne oder als Mischung zudosiert werden. Bevorzugt wird der Katalysator mit dem Phenol oberhalb des oberen Wärmetauschers beziehungsweise der Reaktionszone zudosiert, während das Benzylierungsmittel, je nach relativer Flüchtigkeit bezüglich Phenol (II), direkt unterhalb des oberen Wärmetauschers beziehungsweise im oberen Drittel, in der Mitte beziehungsweise im mittleren Drittel oder am unteren Ende beziehungsweise im unteren Drittel des Reaktionsteils zudosiert wird, je nach dem ob es schwer-, mittel- oder leichtflüchtig ist.

Der Zuspeisepunkt wird so gewählt, daß möglichst kein benzylisches Edukt beziehungsweise Zwischenprodukt den Reaktionsbereich unumgesetzt verläßt; dies gilt insbesondere in Richtung auf den unteren Kolonnenteil. Der optimale Punkt hängt mit der relativen Flüchtigkeit von Phenol (II) und Benzylierungsmittel (III) zusammen und kann durch einfache Experimente und Rechnungen für jeden speziellen Fall leicht ermittelt werden. Verläßt ein besonders leicht flüchtiges Benzylierungsmittel in stärkerem Maße den Reaktionsbereich in Richtung Kolonnenkopf, so kann es leicht, gegebenenfalls vermischt mit nicht abgetrenntem Phenol, dem oberen Kolonnenteil entnommen und zusammen mit frischem Benzylierungsmittel dem unteren Teil des Reaktionsbereichs der Kolonne zugeführt werden.

Ein zusätzlicher Azeotropbildner kann prinzipiell an jeder Stelle der Kolonne eingespeist werden, bevorzugt wird er mit dem Phenol und dem sauren Katalysator gemischt zudosiert.

Er dient zur möglichst leichten und vollständigen Abtrennung des leichtsiedenden Spaltproduktes $H\text{-}R^6$ vom Phenol (II) im oberen Teil der Kolonne.

Das gilt besonders, wenn als Benzylierungsmittel Benzylalkohol oder Dibenzylether verwendet wird und das zu benzylierende Phenol (II) mit Wasser ein Azeotrop bildet und in jedem Verhältnis mischbar ist.

Derartige Azeotropbildner sind dem Fachmann bekannt, beispielsweise seien aufgeführt: Benzol, Toluol, o-Xylol, p-Xylol, Chlorbenzol, Butanol, Hexanol, Diethylether, Dibutylether und Tetrachlorkohlenstoff.

Die Verfahrensweisen zur Separierung von azeotropen Mischungen durch Destillation mittels Hilfskomponenten sind in Chem. Eng. Prog. 85, 63 - 69 (1989) übersichtlich zusammengestellt und an Beispielen erläutert.

Das molare Dosierungsverhältnis von Phenol zu Benzylierungskomponente (in Benzyleinheiten) muß je nach Phenol- und Benzyl-Austrag über Kopf der Destillationskolonne und je nach zusätzlichem Benzyl-Verbrauch durch Bildung von hochsiedenden Nebenprodukten leicht variiert werden, um den internen Phenolüberschuß aufrecht zu erhalten. Es liegt im allgemeinen bei 2:1 bis 1:2, bevorzugt bei 1,5:1 bis 1:1,5,

besonders bevorzugt bei 1,1:1 bis 1:1,1.

Die über Kopf abdestillierten Phenol-, Benzylierungsmittel- und Azeotropbildner-Mengen können, nach der Abtrennung des leichtsiedenden Spaltproduktes, der Destillation wieder zugeführt werden.

Der Druckbereich, in dem das erfindungsgemäße Verfahren durchgeführt wird, liegt zwischen 1 mbar und 10 bar, bevorzugt zwischen 10 mbar und 1 bar, besonders bevorzugt zwischen 20 mbar und 500 mbar.

Die Temperatur im Reaktionsbereich der Kolonne liegt beim erfindungsgemäßen Verfahren zwischen 100 und 240 °C, bevorzugt zwischen 120 und 210 °C, besonders bevorzugt zwischen 140 und 190 °C, ganz besonders bevorzugt zwischen 160 und 180 °C.

Die Temperatur im unteren Teil der Kolonne liegt oberhalb der Temperatur im Reaktionsteil, jedoch unterhalb 350 °C, bevorzugt unterhalb 300 °C, besonders bevorzugt unterhalb 250 °C.

Die Verwendung einer sauren löslichen Verbindung zur Beschleunigung der Reaktion im erfindungsgemäßen Verfahren ist besonders vorteilhaft. Man benötigt zur effektiven Durchführung einer Reaktion in einer Destillationskolonne große Reaktionsgeschwindigkeiten und im Falle eines stationären Katalysatorsystems, einen standfesten heterogenen Kontakt. Eigene Versuche zur Benzylierung von p-Chlorphenol mittels Benzylalkohol oder Benzylchlorid, katalysiert durch stationäre heterogene Katalysatoren in einer Destillationskolonne, ergaben zwar gute Umsätze und Selektivitäten bei allerdings nur unzureichenden Standzeiten der Kontakte, was notwendigerweise immer wieder Unterbrechungen der Produktion und umfangreiche Reinigungs- und Regenerierungsoperationen bedingt.

Es wurde nun gefunden, daß die Verwendung von gelösten sauren Katalysatoren eine störungsfreie Durchführung der Reaktion entsprechend dem erfindungsgemäßen Verfahren gewährleistet.

Als homogene Katalysatoren können Friedel-Crafts-Katalysatoren eingesetzt werden. Dies sind Metallsalze, beziehungsweise -komplexe und Mischungen aus Salzen, beziehungsweise Komplexen und deren Hydrate, Phenolate, Alkohole und Säureadukte mit Kationen der Oxidationsstufen I, II, III, IV und V, wie sie z.B. von G.A. Olah in "Friedel-Crafts and Related Reactions" (Vol. I, S. 284 - 290, S. 307 - 308, S. 314 - 315) aufgelistet worden sind und welche weniger in der Lewis-und mehr in der Brönsted-Säureform aktiv sind (S. 208 - 215).

Bevorzugt werden direkt Brönsted-Säuren als Katalysatoren eingesetzt, wie z.B. von G.A. Olah auf S. 238 aufgelistet. Das sind mittelstark bis stark saure Katalysatoren mit $pK_a$-Werten zwischen plus 3 und minus 12 (relativ zu Wasser, gemessen in $H_2SO_4/H_2O$-Mischungen).

J. Blackwell beschreibt die Durchführung der Reaktion zwischen Benzylbenzolsulfonat und Phenol bei 125 °C binnen mehrerer Stunden (Soc. 1963, S. 366 - 73). In SU 333 863 wird Benzylalkohol mit Phenol in Gegenwart von ungefähr 1,5 % an Hydrogensulfaten ($pK_a$ = 1,99, Bruckenstein und Kolthoff, "Treatise on Analytical Chemistry", Vol. 1, pt. 1, S. 432 - 433) binnen mehrerer Stunden unter Wasserausschleusung umgesetzt. In C.A. 79: 42079 wird die Umsetzung mit 1 bis 2 Mol% $NaHSO_4$ bei 150 °C als optimal beschrieben.

A.R. Abdurasaleva verwendet bei 100 bis 160 °C 10 Gew.-% $H_2SO_4$ ($pK_a$ = -9, Bell, "The Proton in Chemistry", 2nd ed., Cornwell University Press, Ithaca, N.Y., 1973) oder $H_3PO_4$ ($pK_a$ = +0,85) (Zh. Org. Khim., 8 (1972) S. 134 - 36, 7 (1971) S. 1001 - 3).

Der Einsatz von Katalysatormengen um 10 % entspricht demnach dem Stand der Technik und läßt schließen, daß die Benzylierung von Phenolen mit Benzylakoholen oder Benzylchloriden eine nur mäßig schnelle Reaktion ist, welche größere Mengen an Katalysator benötigt. Hierbei scheint eine Tendenz zu bestehen, schwächere Säuren wie z.B. Hydrogensulfate einzusetzen. Dem entspricht auf der Seite der heterogenen Katalysatoren der Einsatz von Na-Y zur Benzylierung von p-substituierten Phenolen (EP 278 211).

In Abwesenheit von Katalysatoren wird nach eigenen Versuchen selbst nach 8 Stunden bei 180 °C in einer Mischung aus p-Chlorphenol und Benzylalkohol kein Benzylierungsprodukt nachgewiesen (Vergleichsversuch 1).

Für die effektive Durchführung einer Reaktion in einer Destillationskolonne ist es wünschenswert, Reaktionszeiten im Bereich deutlich unterhalb einer Stunde, am besten von wenigen Minuten zu realisieren.

Aufgrund der o.a. Literatur war zu erwarten, daß für das erfindungsgemäß durchzuführende Verfahren noch größere Mengen an Katalysator benötigt würden, als es für das bekannte Batch-Verfahren mit geringer Raumzeitausbeute und langer Verweilzeit (Reaktionszeit) beschrieben ist. Stark saure Katalysatoren im Sinne obiger Aufzählung, wie sie im erfindungsgemäßen Verfahren eingesetzt werden, sind nach dem Stand der Technik entsprechend obigen Zitaten als weniger vorteilhaft einzustufen (siehe Verwendung von Hydrogensulfaten und von Na-Y).

Überraschenderweise wurde gefunden, daß schon geringe Mengen einer starken Säure mit $pK_a$-Werten von 0 bis -12 die gewünschte Phenolbenzylierung enorm beschleunigen.

So ist die Reaktion zwischen p-Chlorphenol und Benzylalkohol in Anwesenheit von z.B. 0,1 - 1 Mol% Methansulfonsäure, Schwefelsäure, Toluolsulfonsäure oder Benzolsulfonsäure bei 160 °C binnen 15 Minuten quantitativ abgelaufen, so daß weder Benzylalkohol noch Zwischenprodukte wie Dibenzylether und p-Chlorphenyl-benzylether nachzuweisen sind.

Schwächere Säure wie z.B. Essigsäure und Benzoesäure, müssen in wesentlich größeren Mengen von mindestens 5 Mol% eingesetzt werden und erreichen auch dann bei weitem nicht die Katalysegeschwindigkeit von starken Säuren im Sinne obiger Definition.

Diese Beobachtung ist speziell für das erfindungsgemäße Verfahren von großer Bedeutung, da es nicht nötig ist, zur Erzielung der gewünschten Reaktionsgeschwindigkeiten große Katalysatormengen im Kreis zu führen, es kann sogar gegebenenfalls auf eine Wiederverwendung des Katalysators verzichtet werden.

In einer besonderen Durchführungsform des erfindungsgemäßen Verfahrens kann er sogar nach Verlassen des Reaktionsteils noch in der App. durch Einspeisen einer entsprechenden Menge einer nichtflüchtigen Base neutralisiert werden, um gegebenenfalls unerwünschten Nebenreaktionen im unteren Kolonnenteil zu unterbinden.

Besonders bevorzugt wird das erfindungsgemäße Verfahren demnach in Gegenwart von löslichen Brönsted-Säuren durchgeführt, deren $pK_a$-Wert (gemessen in $H_2SO_4/H_2O$-Mischungen, relativ zu Wasser) zwischen 0 und -12, bevorzugt zwischen -1 und -12, besonders bevorzugt zwischen -2 und -12 liegt.

Die molare Menge an Säure, relativ zu zudosierten Benzyläquivalenten liegt zwischen 10 und 0,001 Mol%, bevorzugt zwischen 5 und 0,002 Mol%, besonders bevorzugt zwischen 1 und 0,004 Mol%.

Der Katalysator gerät nach Durchlaufen der Reaktionszone, in der er je nach Flüchtigkeit mehr oder weniger angereichert oder verdünnt wird, in den unteren Aufarbeitungsteil der Apparatur, dort kann er, insbesondere bei sehr hohen Temperaturen, zu Zersetzungsreaktionen Anlaß geben und das Produkt verunreinigen.

Daher kann das erfindungsgemäße Verfahren gegebenenfalls in der Weise modifiziert werden, daß unterhalb der Reaktionszone, beziehungsweise des mittleren Wärmetauschers und oberhalb der Produktentnahmestelle eine Base oder eine Puffermischung zur Neutralisierung oder Abstumpfung des herabfließenden Produktstromes zudosiert wird.

Der pH-Wert des Produktstromes soll nach Zumischung von Base größer 2, bevorzugt größer 3, besonders bevorzugt größer 4 sein. Als Basen (beziehungsweise Puffersysteme) geeignet sind nicht flüchtige anorganische und organische Basen, wie z.B. Phenolate, Carbonate, Hydrogencarbonate, Carboxylate, Alkoholate, Phosphate, Hydrogenphosphate, Dihydrogenphosphate, Sulfate und Hydroxide oder Oxide von Alkali- und Erdalkalimetallen.

Die Dosierung der Base erfolgt parallel zur Säuredosierung und kann nach bekannten Methoden gemessen und gesteuert werden.

Die erzielbaren Selektivitäten beim erfindungsgemäßen Verfahren hängen vom internen Phenolüberschuß ab und liegen i,a. über 90 % d. Th.

Das erfindungsgemäße Verfahren stellt einen deutlichen Fortschritt im Vergleich zum Stand der Technik dar, weil es einen selektiven kontinuierlichen Zugang zu o-benzylierten, p-substituierten Phenolen eröffnet bei einfacher Apparatur, guter Energie- und Stoffausnutzung und hoher Raumzeitausbeute.

Daß nur sehr geringe Mengen einer starken Säure im erfindungsgemäßen Verfahren benötigt werden, stellt einen weiteren deutlichen Fortschritt dar.

p-substituierte o-Benzylphenole sind bekannte Verbindungen und werden nach DE-OS 22 11 266 und US-PS 45 14 577 als Antioxdantien und Bakterizide eingesetzt.

## Beispiele

### Beispiel 1 (Vergleichsbeispiel)

Rührversuch, ohne Katalysator

3000 g einer Mischung aus p-Chlorphenol und Benzylalkohol (Molverhältnis 5 zu 1) wurden bei Raumtemperatur binnen 30 Minuten über eine 10 cm hohe $KHCO_3$-Schüttung gegeben (ø = 3 cm). Die so erhaltene säurefreie Eduktmischung wurde unter einer Stickstoffatmosphäre aufbewahrt.

100 g der Eduktmischung wurden unter Stickstoff auf 180 °C erwärmt. Nach 8 Stunden entnahm man eine kleine Probe und führte eine gaschromatographische Analyse durch. Es war kein Produkt oder Zwischenprodukt nachweisbar. (Nachweisgrenze ca. 0,01 Massenproz.)

Beispiel 2 (Vergleichsbeispiel)
Rührversuche, verschiedene Säurekatalysatoren

100 g der in Beispiel 1 über $KHCO_3$ vorbereiteten Eduktmischung wurden mit 0,1, beziehungsweise 1, beziehungsweise 5 Mol% (bezogen auf die Summe der Phenol- und Benzylalkohol-Molmengen) einer Säure gemischt und unter einem leichten Stickstoffstrom auf 160 °C erwärmt. Der Gehalt an 2-Benzyl-4-Chlorphenol in Flächenprozent wird in Tab. 1 in Abhängigkeit von der Zeit angegeben. Flächenprozente sind die aus der gaschromatographischen Analyse.

Tabelle 1

| | |
|---|---|
| | 2-Benzyl-4-Chlorphenol-Bildung in Abhängigkeit vom Säuregehalt und von der Zeit (Gehalt in Fl.-%).<br>$pK_a$-Werte der Säuren, sofern angegeben, nach E.P. Serjeant und B. Dempsey, "Ionisation Constants of Organic Acids in Aqueous Solution", Pergamon, New York 1979. |
| A | = keine Säure ($pK_a$ = -) |
| B | = Oxalsäure (1,0 - 1,295) |
| C | = Essigsäure (4,48 - 4,76) |
| D | = Methansulfonsäure (-1,2 bis -1,86) |
| E | = Benzoesäure (4,205) |
| F | = Benzolsulfonsäure (-) |
| G | = Pivalinsäue (-) |
| H | = Trifluoressigsäure (0,50 - 0,52) |
| I | = Trichloressigsäure (0,517 - 0,538) |
| J | = Chlordifluoressigsäure (-) |
| K | = Schwefelsäure (-) |
| L | = Phosphorsäure (-) |

Tabelle 1 (Fortsetzung)

| Zeit Min. | mol % | A | B | C | D | E | F | G | H | I | J | K | L |
|---|---|---|---|---|---|---|---|---|---|---|---|---|---|
| | 0,1 | 0 | 0 | 0 | 0,0 | 0 | 0 | 0 | 0 | 0 | 0 | 0 | 0 |
| 5 | 1 | 0 | 1,2 | 0 | 22,7 | 0 | 23,5 | 0 | 0 | 0 | 0,1 | 21,0 | - |
| | 5 | 0 | 7,1 | 0 | 22,7 | 0 | 23,6 | 0 | 1,0 | 1,2 | 3,2 | 22,3 | 2,7 |
| | 0,1 | 0 | 0 | 0 | 0,0 | 0 | 0 | 0 | 0 | 0 | 0 | 0 | 0 |
| 15 | 1 | 0 | 3,5 | 0 | 20,7 | 0 | 23,4 | 0 | 0 | 0 | 0,2 | 21,1 | - |
| | 5 | 0 | 12,8 | 0 | 22,8 | 0 | 23,6 | 0 | 4,2 | 4,9 | 6,6 | 21,8 | 10,1 |
| | 0,1 | 0 | 0 | 0 | 0,0 | 0 | 0 | 0 | 0 | 0 | 0 | 0 | 0 |
| 60 | 1 | 0 | 13,6 | 0 | 22,7 | 0 | 23,4 | 0 | 0 | 0 | 2,9 | 21,1 | - |
| | 5 | 0 | 22,5 | 0 | 22,9 | 0 | 23,6 | 0 | 15,8 | 16,7 | 14,9 | 21,8 | 20,7 |
| | 0,1 | 0 | 0 | 0 | 0,0 | 0 | 0 | 0 | 0 | 0 | 0 | 0 | 0 |
| 120 | 1 | 0 | 22,8 | 0 | 22,8 | 0 | 23,4 | 0 | 0 | 0 | 22,1 | 21,2 | - |
| | 5 | 0 | 22,4 | 0 | 22,8 | 0 | 23,5 | 0 | 21,7 | 21,6 | 21,5 | 21,8 | 21,2 |

22 - 24 % 2-Benzyl-4-Chlorphenol entspricht 100 % Umsatz

Beispiel 3 (Vergleichsbeispiel)
Reaktionsdestillation, Katalysator Zeolith Na-Y, Edukte: Benzylalkohol, p-Chlorphenol

Die App. bestand von unten nach oben aus:
Glaskolben (250 ml) mit geregelter Stickstoffeinleitung zum Aufnehmen des Sumpfproduktes (aus diesem Kolben wird das Hauptprodukt der Umsetzung gewonnen, indem es von Zeit zu Zeit abgesaugt wird);
Ölthermostatisierter Schlangenverdampfer (Schlangenlänge ca. 1,50 m, Rohrinnendurchmesser ca. 1,5 cm);
vakuumverspiegelte Füllkörperkolonne, gefüllt mit 0,5 cm großen Porzellansattelkörpern (Innendurchmesser der Kolonne ca. 2,7 cm, Höhe der Kolonne ca. 35 cm);
Ölthermostatisierer Kolonnenteil, gefüllt mit Porzellansattelkörpern (Durchmesser ca. 2,7 cm, Höhe ca. 35 cm); zwei Füllkörperkolonnen (vakuumverspiegelt) mit Katalysator (Reaktionsteil, Durchmesser ca. 2,7 cm, Höhe ca. 35 cm, Gesamthöhe ca. 70 cm);
wassergekühlte Eindosierstelle (Eduktmischung wird bis zur Austropföffnung gekühlt, um eine vorzeitige Umsetzung zu verhindern);
Ölthermostatisierter Kolonnenteil mit Porzellansattelkörpern (Durchmesser ca. 2,7 cm, Höhe ca. 15 cm);
Luftbrücke zum abwärtsbetriebenen Kondensationsteil aus zwei Intensivkühlern, Anschützaufsatz, Vakuumentnahmestutzen und Gaskappe mit Vakuumschlauch.

Das Vakuum in der App. wurde durch eine magnetgesteuerte Wasserstrahlpumpe konstant gehalten (± 1,5 mbar).

Die Eduktgemischdosierung erfolgte durch eine vakuumdichte Dosierpumpe (TELAB, PTFE-Minidosierer). Die drei thermostatisierten Kolonnenbereiche wurden mittels Ölthermostaten beheizt, beziehungsweise gekühlt.

In den Reaktionsteil der Apparatur wurden 190 g Zeolith Na-Y (Modul = 4,7), gebunden mit 30 % $Al_2O_3$ (Pural SCF), in Form 0,5 bis 2,5 cm langer Stäbchen mit einem Durchmesser von 2 - 3 mm gegeben.

Der Druck wurde auf 120 mbar eingestellt und zuerst reines p-Chlorphenol aus einem beheizten Tropftrichter in den unteren Teil der Apparatur gegeben, bis ein deutlicher Rückfluß in der gesamten Apparatur eingestellt war (Öltemperatur unterer Verdampfer ca. 260 °C, Öltemperatur mittlerer Verdampfer ca. 170 °C, Öltemperatur oberer Kondensator ca. 50 °C). Es wurde ein $N_2$-Strom von 0,1 Nl/Std. in den unteren Teil der Apparatur gegeben. Nun wurde eine Mischung aus 1,5 Molen p-Chlorphenol und 1 Mol Benzylalkohol zudosiert (ca. 150 g/Std.). Dabei wurde die Temperatur des unteren Verdampfers hinaufgesetzt, bis eine Temperatur von ca. 190 - 220 °C am Ende der unteren Kolonne gemessen wurde. Die Temperatur des mittleren Verdampfers wurde solange heraufgesetzt, bis ein deutlicher Temperatursprung zwischen dem unteren Ende des Katalysatorbettes und dem oberen Ende der unteren Kolonne bestand (von ca. 150 °C auf ca. 170 °C). Die Temperatur des oberen Kondensators blieb auf 50 °C gestellt und wurde nur bei drohender Kolonnenflutung heraufgesetzt.

Die Temperatur oberhalb des Kondensators (Dephlegmator) schwankte zwischen 120 und 140 °C.

In den Sumpfkolben der Apparatur tropften pro Stunde ca. 105 bis 106 g einer Mischung aus 97,8 % 2-Benzyl-4-Chlorphenol, 0,8 % Benzyl-4-Chlorphenylether und 1,4 % Hochsiedern (mehrfachbenzylierte Phenole). Nach Durchlaufen der Luftbrücke kondensierten im Kondensationsteil der Apparatur 44 - 45 g einer Mischung aus Wasser, Benzylalkohol und p-Chlorphenol. Das Verhältnis Benzylalkohol zu p-Chlorphenol in der organischen Phase betrug 5,4 % zu 94,6 %.

Mit zunehmender Laufzeit stieg die Menge an Kondensat unter Zunahme des Benzylalkoholanteils ständig an, ein Ausdruck der fortschreitenden Katalysatordesaktivierung, welche sich in einer dunkelbraunen Verfärbung der Katalysatorteilchen zeigte.

Nach 100 Stunden war der Katalysator fast völlig desaktiviert.

Beispiel 4 (Vergleichsbeispiel)
Reaktionsdestillation, ohne Katalysator, Edukte: Benzylchlorid, p-Chlorphenol

In der in Beispiel 3 beschriebenen Apparatur wurde der Katalysator durch inerte Porzellansattelkörper ersetzt. Statt Benzylalkohol wurde Benzylchlorid zudosiert, sonst wurde wie im Beispiel 3 verfahren.

Der weitaus größte Teil der Eduktmischung destillierte unumgesetzt ab. Das Sumpfprodukt bestand aus 2-Benzyl-4-Chlorphenol und Benzyl-4-Chlorphenylether im Verhältnis 0,7:1.

Der Anteil hochsiedender Produkte im Sumpf lag bei 7 %.

Beispiel 5 (Vergleichsbeispiel)
Wie Beispiel 4, jedoch HCl-Gegenstrom

Das Beispiel 5 unterschied sich vom Beispiel 4, indem 0,1 Nl HCl-Gas/Stunde in den unteren Kolben eingespeist wurden. Das Ergebnis des Beispiels entsprach dem in Beispiel 4 ohne HCl-Gegenstrom.

Beispiel 6 (Vergleichsbeispiel)
Reaktionsdestillation, Katalysator saures Schichtsilicat, Edukte: Benzylchlorid, p-Chlorphenol

In der in Beispiel 3 beschriebenen Apparatur wurde der Katalysator durch Raschigringe aus K306 (saures Schichtsilicat der Fa. Südchemie) ersetzt. Ansonsten wurde wie in Beispiel 4 verfahren.

Das Ergebnis entsprach dem des Beispiels 4, jedoch enthielt das Produkt keinen Benzyl-4-Chlorphenylether und bestand zu 80 % aus 2-Benzyl-4-Chlorphenol und zu 20 % aus Hochsiedern.

Beispiel 7 (Vergleichsbeispiel)
ähnlich Beispiel 6

Das Beispiel 6 wurde wiederholt, indem das Benzylchlorid getrennt vom p-Chlorphenol 35 cm oberhalb des unteren Katalysatorendes eingespeist wurde.

Bei einer Belastung von ca. 106 g/h p-Chlorphenol (Pumpe und Zuleitung beheizt) und ca. 90 g/h Benzylchlorid erhielt man ca. 152 g/h einer Mischung aus 95,2 % 2-Benzyl-4-chlorphenpl und 4 % Hochsiedern. Als Destillat wurden ca. 18 g/h p-Chlorphenol erhalten.

Der Katalysator desaktivierte binnen 2 Stunden.

Beispiel 8
Reaktionsdestillation, Katalysator Methansulfonsäure, Edukte: Benzylalkohol, p-Chlorphenol

In der in Beispiel 3 beschriebenen Apparatur wurde der Katalysator durch inerte Porzellansattelträger ersetzt. Die Apparatur wurde wieder wie beschrieben bei 120 mbar zuerst mit reinem p-Chlorphenol angefahren und von einem kleinen $N_2$-Gegenstrom durchströmt (0,11 Nl/Std.).

Sodann wurde begonnen, ca. 100 g/h einer Mischung aus 128,5 g p-Chlorphenol, 108 g Benzylalkohol und 192 mg Methansulfonsäure (0,2 Mol%) über den gekühlten Zutropffinger in die Apparatur zu pumpen. Der untere Thermostat und der mittlere Thermostat pumpten Öl mit einer Temperatur von 250 ° C, der obere mit 67 ° C im Kreis.

Am Ende der untersten Kolonne stellte sich eine Temperatur von 190 - 200 ° C ein, am mittleren Verdampfer wurde ein Temperatursprung von 152 - 154 auf 165 - 195 ° C gemessen.

Das untere Ende der Apparatur verließen pro Stunde ca. 84,1 bis 89,8 g einer Mischung aus 92 - 97 Gew.-% 2-Benzyl-4-Chlorphenol, 1 % 3-Benzyl-4-Chlorphenol und 2 - 7 % Hochsiedern. Benzyl-4-Chlorphenylether war nicht nachzuweisen.

Am Kolonnenkopf fielen pro Stunde ca. 20,2 bis 15,9 g einer Mischung, bestehend aus ca. 7,6 g Wasser und p-Chlorphenol, an.

Die Umsetzung lief, abgesehen von apparativ bedingten leichten Schwankungen, konstant ab.

Beispiel 9
Reaktionsdestillation, Katalysator Methansulfonsäure, Edukte: Benzylalkohol, p-Chlorphenol

Wie Beispiel 8, jedoch Dosierleistung ca. 65 g/Std. einer Mischung aus 128,5 g p-Chlorphenol, 108 g Benzylalkohol und 19 mg Methansulfonsäure (0,02 Mol%). Das Ergebnis entsprach dem von Beispiel 8, jedoch wurden 0,1 % Benzyl-4-Chlorphenylether im unteren Produktstrom gefunden.

Beispiel 10
Reaktionsdestillation, Katalysator Schwefelsäure, Edukte: Benzylalkohol, p-Chlorphenol

Wie Beispiel 8, jedoch Dosierleistung ca. 100 g/Std. einer Mischung aus 128,5 g p-Chlorphenol, 108 g Benzylalkohol und 200 mg Schwefelsäure (100%ig).

Das Ergebnis war identisch mit dem von Beispiel 8 mit dem Unterschied, daß das Sumpfprodukt nicht in schwach gelblich bis bräunlich gefärbter Form, sondern intensiv grün gefärbt anfiel.

Beispiel 11

Reaktionsdestillation, Katalysator Schwefelsäure, Edukte: Benzylalkohol, p-Chlorphenol

Wie Beispiel 10, jedoch wurde direkt unterhalb des mittleren Verdampferteils 144 mg/Std. $NaHCO_3$ in 10 g Produktmischung zudosiert.

Das Ergebnis war identisch mit dem von Beispiel 10 mit dem Unterschied, daß das Sumpfprodukt braun gefärbt war und eine Trübung durch ausgefallenes Natriumsalz aufwies.

Beispiel 12

Reaktionsdestillation, Katalysator Methansulfonsäure, Edukte: Benzylchlorid, p-Chlorphenol

Die Apparatur bestand von unten nach oben aus:
Glaskolben (250 ml) mit geregelter Stickstoffeinleitung zum Aufnehmen des Sumpfproduktes (aus diesem Kolben wurde das Hauptprodukt der Umsetzung gewonnen, indem von Zeit zu Zeit abgesaugt wurde);
Ölthermostatisierter Schlangenverdampfer (Schlangenlänge ca. 1,50 m, Rohrinnendurchmesser ca. 1,5 cm);
vakuumverspiegelte Füllkörperkolonne, gefüllt mit 0,5 cm großen Porzellansattelkörpern (Innendurchmesser der Kolonne ca. 2,7 cm, Höhe der Kolonne ca. 35 cm);
ölthermostatisierter Kolonnenteil, gefüllt mit Porzellansattelkörpern (Durchmesser ca. 2,7 cm, Höhe ca. 35 cm);
Glasrohr mit beheizter Abzweigung zur Zudosierung von Benzylchlorid und zur Rückspeisung von nicht umgesetzter Benzylchlorid/p-Chlorphenol-Mischung (Destillat) (Durchmesser ca. 2,7 cm, Höhe ca. 10 cm; 20 cm Zulaufrohr, mit Heizband auf 200 °C thermostatisiert, Innendurchmesser 1,5 cm);
zur Wärmeisolierung ölthermostatisierte Siebbodenkolonne (10 Böden, Flüssigkeitsfüllung ca. 60 ml, Höhe ca. 60 cm, Innendurchmesser ca. 5 cm);
Glasrohr mit Abzweigung zur Zudosierung von p-Chlorphenol-Katalysatormischung (Durchmesser ca. 2,7 cm, Höhe ca. 10 cm);
ölthermostatisierter Kolonnenteil mit Porzellansattelkörpern (Durchmesser ca. 2,7 cm, Höhe ca. 15 cm);
vakuumverspiegelte Füllkörperkolonne mit Porzellansattelkörpern (Innendurchmesser der Kolonne ca. 2,7 cm, Höhe ca. 30 cm);
Luftbrücke zum abwärtsbetriebenen Kondensationsteil aus zwei Intensivkühlern, Anschützaufsatz, Glaskappe mit Vakuumschlauch und einem isolierten 25 ml Auffangzylinder mit Hahn, aus dem das Kondensat mit definierter Dosierung zur Benzylchlorid-Einspeisung zurückgeführt wurde.

Das Vakuum in der Apparatur wurde durch eine magnetgesteuerte Wasserstrahlpumpe konstant gehalten (± 1,5 mbar).

Die Benzylchlorid-Dosierung erfolgte durch eine vakuumdichte Dosierpumpe (TELAB, PTFE-Minidosierer).

Die Dosierung der p-Chlorphenol/Katalysator-Mischung erfolgte mittels einer beheizten Lewa-Pumpe mit Druckhalteventil (3 bar).

Die Rückführung des Kondensates erfolgte mit einer vakuumdichten Dosierpumpe (TELAB, PTFE-Minidosierer).

In die beheizte Vorlage der Lewa-Pumpe wurde eine Mischung aus 1000 g p-Chlorphenol und 800 mg Methansulfonsäure-Hydrat gegeben, die Benzylchloridvorlage gefüllt und der Druck der Apparatur auf 120 mbar eingestellt.
Daraufhin wurde begonnen, p-Chlorphenol zuzupumpen, bis ein deutlicher Rückfluß in der Apparatur eingestellt war. (Öltemperatur unterer Verdampfer ca. 250 °C, Öltemperatur mittlerer Verdampfer ca. 238 °C, Öltemperatur Siebbodenkolonne ca. 142 °C, Öltemperatur oberer Kondensator ca. 60 °C).
Der Gesamtflüssigkeitsinhalt der Destillationsapparatur war zu diesem Zeitpunkt ungefähr 80 ml.
Es wurde ein $N_2$-Strom von 0,1 Nl/Std. in den unteren Teil der Apparatur gegeben.
Benzylchlorid und p-Chlorphenyldosierung wurden auf jeweils 82 ml/Std. eingestellt.
Am Kopf der Apparatur stellte sich eine Temperatur von 100 bis 105 °C ein, und eine Mischung aus 67 Gew.-% Benzylchlorid und 33 Gew.-% p-Chlorphenol kondensierte und floß in die Vorlage der Recyclisierungspumpe. Diese wurde auf ca. 400 ml/Std. eingestellt und speiste das Kondensat über die Benzylchlorid-Einspeisung zurück in die Kolonne.
In den Sumpfkolben der Apparatur tropften pro Stunde ca. 147 bis 149 g einer Mischung aus 90 Gew.-% 2-Benzyl-4-chlorphenol, 1 Gew.-% 3-Benzyl-4-chlorphenol und 9 Gew.-% Hochsiedern (mehrfach benzylierte Phenole).
Der Gehalt an Benzyl-4-chlorphenylether war kleiner als 0,01 %.

Beispiel 13

Reaktionsdestillation, Katalysator Methansulfonsäure, Edukte: Benzylchlorid, p-Chlorphenol

Wie Beispiel 12, jedoch wurden 27 ml Benzylchlorid und 23 ml p-Chlorphenol pro Stunde zudosiert.

Bei 90 bis 92 °C kondensierte am Kopf der Apparatur eine Mischung aus 74 Gew.-% Benzylchlorid und 26 Gew.-% p-Chlorphenol. Diese Mischung wurde mit 100 ml/Std. in den Reaktor zurückgepumpt. In den Sumpfkolben der Apparatur tropften pro Stunde ca. 50 g einer Mischung aus 95 % 2-Benzyl-4-chlorphenol, 1 % 3-Benzyl-4-chlorphenol und 4 % Hochsiedern. Benzyl-4-chlorphenylether war nicht nachweisbar.

## Patentansprüche

**1.** Verfahren zur Herstellung von in p-Stellung substituierten o-Benzylphenolen der Formel

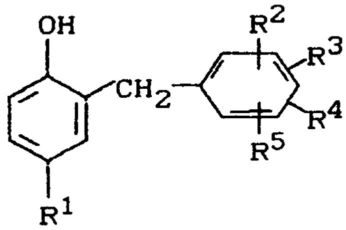

in der

$R^1$ für Halogen, Hydroxy, $C_1$-$C_{12}$-Alkyl, $C_1$-$C_{12}$-Alkoxy, $C_1$-$C_{12}$-Alkylmercapto, $C_6$-$C_{12}$-Aryl, $C_6$-$C_{12}$-Aryloxy oder $C_6$-$C_{12}$-Arylmercapto steht und

$R^2$ bis $R^5$ unabhängig voneinander Wasserstoff, Halogen, die Cyanogruppe, $C_1$-$C_{12}$-Alkyl, $C_1$-$C_{12}$-Alkoxy, $C_1$-$C_{12}$-Alkylmercapto, $C_6$-$C_{12}$-Aryl, $C_6$-$C_{12}$-Aryloxy oder $C_6$-$C_{12}$-Arylmercapto bedeuten und zwei der Reste $R^2$ bis $R^5$ Trimethylen, Tetramethylen oder den Rest eines ankondensierten Benzolringes bedeuten,

durch Umsetzung von p-substituierten Phenolen der Formel

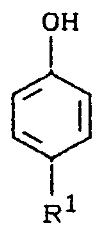

,

in der

$R^1$ die oben genannte Bedeutung besitzt,

mit Benzylverbindungen der Formel

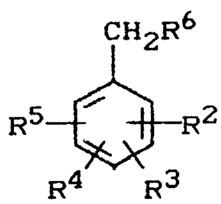

,

in der

$R^2$ bis $R^5$ die oben genannte Bedeutung besitzen und

$R^6$ für Halogen, $C_1$-$C_{12}$-Alkylcarboxy, substituiertes oder nicht substituiertes Phenylcarboxy, $C_1$-$C_{12}$-Alkyl- oder Phenylsulfatoxy, Hydroxy, $C_1$-$C_{12}$-Alkoxy, substituiertes oder nichtsubstituiertes Benzyloxy oder substituiertes oder nichtsubstituiertes p-$R^1$-Phenoxy,

Trichloracetyloxy oder Methansulfonyloxy steht, wobei $R^1$ die oben genannte Bedeutung besitzt,

dadurch gekennzeichnet, daß die Umsetzung in einer kontinuierlich arbeitenden Destillationsapparatur in Gegenwart mindestens eines löslichen sauren Katalysators in einer Menge zwischen 10 und 0,001 Mol-%, bezogen auf Äquivalente an zudosierter Benzylverbindung, durchgeführt wird, wobei in einem Druckbereich zwischen 1 mbar und 10 bar und in einem Temperaturbereich zwischen 100 und 240 °C im Reaktionsteil der Kolonne und in einem Temperaturbereich oberhalb desjenigen im Reaktionsteil, aber unterhalb von 350 °C im unteren Teil der Kolonne gearbeitet wird.

**2.** Verfahren nach Anspruch 1, dadurch gekennzeichnet, daß reaktorintern ein Phenolüberschuß aufrecht erhalten wird.

**3.** Verfahren nach Anspruch 1, dadurch gekennzeichnet, daß die Destillationsapparatur im mittleren Bereich einen zusätzlichen Wärmetauscher zur Verdampfung des größten Teils des internen Phenolüberschusses besitzt.

**4.** Verfahren nach Anspruch 1, dadurch gekennzeichnet, daß die Destillationsapparatur im oberen Bereich einen zusätzlichen Wärmetauscher zur Niederschlagung des größten Teils des internen Phenolüberschusses besitzt.

**5.** Verfahren nach Anspruch 1, dadurch gekennzeichnet, daß zur wirkungsvollen Trennung der Edukte vom leichtflüchtigen Reaktionsprodukt ein zusätzlicher Azeotropbildner eingesetzt wird.

**6.** Verfahren nach Anspruch 1, dadurch gekennzeichnet, daß eine der eingesetzten katalytisch wirksamen Säuren einen $pK_a < 0$ besitzt.

**7.** Verfahren nach Anspruch 1, dadurch gekennzeichnet, daß eine der katalytisch wirksamen Säuren Toluolsulfonsäure, Benzolsulfonsäure, Schwefelsäure oder Methansulfonsäure ist.

**8.** Verfahren nach Anspruch 1, dadurch gekennzeichnet, daß die Menge an Säure relativ zum eingesetzten Benzyläquivalent zwischen 5 und 0,02 Mol% liegt.

**9.** Verfahren nach Anspruch 1, dadurch gekennzeichnet, daß das Verfahren in einem Druckbereich von 10 mbar bis 1 bar durchgeführt wird.

**10.** Verfahren nach Anspruch 1, dadurch gekennzeichnet, daß im Umsetzungsbereich der Kolonne das Verhältnis an Phenolkomponente zur Summe aus Produktkomponente und unumgesetzten Benzyläquivalenten an jeder Stelle größer als 2 ist.

## Claims

**1.** Process for the preparation of o-benzylphenols substituted in the p-position, of the formula

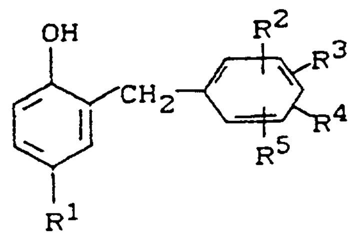

in which

$R^1$ represents halogen, hydroxyl, $C_1$-$C_{12}$-alkyl, $C_1$-$C_{12}$-alkoxy, $C_1$-$C_{12}$-alkylmercapto, $C_6$-$C_{12}$-aryl, $C_6$-$C_{12}$-aryloxy or $C_6$-$C_{12}$-arylmercapto and

$R^2$ to $R^5$ independently of one another denote hydrogen, halogen, the cyano group, $C_1$-$C_{12}$-alkyl, $C_1$-$C_{12}$-alkoxy, $C_1$-$C_{12}$-alkylmercapto, $C_6$-$C_{12}$-aryl, $C_6$-$C_{12}$-aryloxy or $C_6$-$C_{12}$-arylmercapto, and two of the radicals $R^2$ to $R^5$ denote trimethylene, tetramethylene or the

radical of a fused-on benzene ring,

by reaction of p-substituted phenols of the formula

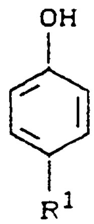

,

in which

$R^1$ has the abovementioned meaning,

with benzyl compounds of the formula

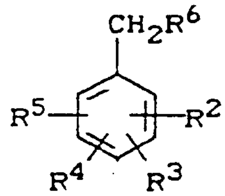

,

in which

$R^2$ to $R^5$ have the abovementioned meaning and

$R^6$ represents halogen, $C_1$-$C_{12}$-alkylcarboxyl, substituted or unsubstituted phenylcarboxyl, $C_1$-$C_{12}$-alkyl- or phenylsulphatoxy, hydroxyl, $C_1$-$C_{12}$-alkoxy, substituted or unsubstituted benzyloxy or substituted or unsubstituted p-$R^1$-phenoxy, trichloroacetyloxy or methanesulphonyloxy, wherein $R^1$ has the abovementioned meaning,

characterized in that the reaction is carried out in a continuously operating distillation apparatus in the presence of at least one soluble acid catalyst in an amount of between 10 and 0.001 mol %, based on equivalents of benzyl compound metered in, the process being carried out in a pressure range between 1 mbar and 10 bar and in a temperature range between 100 and 240 °C in the reaction part of the column and in a temperature range above that in the reaction part, but below 350 °C, in the lower part of the column.

2. Process according to Claim 1, characterized in that an excess of phenol is maintained inside the reactor.

3. Process according to Claim 1, characterized in that the distillation apparatus has, in the central region, an additional heat exchanger for evaporation of the majority of the internal excess of phenol.

4. Process according to Claim 1, characterized in that the distillation apparatus has, in the upper region, an additional heat exchanger for precipitation of the majority of the internal excess of phenol.

5. Process according to Claim 1, characterized in that an additional azeotrope-forming agent is employed for effective separation of the starting materials from the highly volatile reaction product.

6. Process according to Claim 1, characterized in that one of the catalytically active acids employed has a $pK_a$ of < 0.

7. Process according to Claim 1, characterized in that one of the catalytically active acids is toluenesulphonic acid, benzenesulphonic acid, sulphuric acid or methanesulphonic acid.

8. Process according to Claim 1, characterized in that the amount of acid in relation to the benzyl equivalent employed is between 5 and 0.02 mol %.

9. Process according to Claim 1, characterized in that the process is carried out in a pressure range from 10 mbar to 1 bar.

**10.** Process according to Claim 1, characterized in that in the reaction region of the column, the ratio of the phenol component to the sum of the product component and unreacted benzyl equivalents at each point is greater than 2.

## Revendications

**1.** Procédé de préparation d'o-benzylphénols substitués en position p et répondant à la formule :

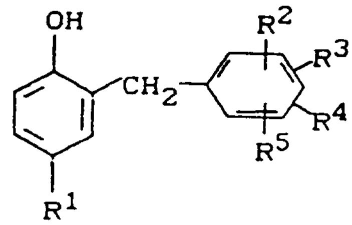

dans laquelle

$R^1$ représente un atome d'halogène, un groupe hydroxyle, alkyle en $C_1$ à $C_{12}$, alcoxy en $C_1$ à $C_{12}$, alkylmercapto en $C_1$ à $C_{12}$, aryle en $C_6$ à $C_{12}$, aryloxy en $C_6$ à $C_{12}$ et arylmercapto en $C_6$ à $C_{12}$, et

$R^2$ à $R^5$ représentent chacun, indépendamment l'un de l'autre, un atome d'hydrogène, un atome d'halogène, le groupe cyano, un groupe alkyle en $C_1$ à $C_{12}$, alcoxy en $C_1$ à $C_{12}$, alkylmercapto en $C_1$ à $C_{12}$, aryle en $C_6$ à $C_{12}$, aryloxy en $C_6$ à $C_{12}$ ou arylmercapto en $C_6$ à $C_{12}$, et deux des restes $R^2$ à $R^5$ peuvent former un groupe triméthylène, tétraméthylène ou le reste d'un noyau benzénique condensé,

par réaction de phénols substitués en para et répondant à la formule :

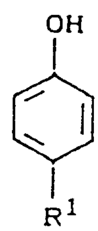
,

dans laquelle

$R^1$ a le sens indiqué ci-dessus,

avec des composés benzyliques de formule :

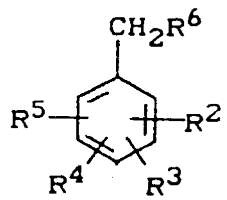
,

dans laquelle

$R^2$ à $R^5$ ont le sens indiqué ci-dessus, et

$R^6$ représente un atome d'halogène, un groupe alkyl(en $C_1$ à $C_{12}$)carboxy , un groupe phénylcarboxy substitué ou non substitué, alkyl(en $C_1$ à $C_{12}$)sulfatoxy ou phénylsulfatoxy, hydroxyle, alcoxy en $C_1$ à $C_{12}$, benzyloxy substitué ou non substitué ou p-$R^1$-phénoxy substitué ou non substitué, trichloracétyloxy ou méthanesulfonyloxy, $R^1$ ayant le sens indiqué ci-dessus,

procédé caractérisé en ce qu'on effectue la réaction dans un appareillage de distillation fonctionnant en continu, en opérant en présence d'au moins un catalyseur acide soluble présent en une quantité comprise entre 10 et 0,01 mol%, par rapport aux équivalents du composé benzylique à introduire, et

l'on travaille dans un intervalle de la pression compris entre 1 mbar et 10 mbar et dans un intervalle de la température compris entre 100 et 240 ° C dans la partie de la colonne consacrée à la réaction et dans un intervalle de la température supérieur à celui régnant dans la partie consacrée à la réaction, mais au-dessous de 350 ° C dans la partie inférieure de la colonne.

**2.** Procédé selon la revendication 1, caractérisé en ce qu'on maintient un excès de phénol à l'intérieur du réacteur.

**3.** Procédé selon la revendication 1, caractérisé en ce que l'appareillage de distillation comporte dans sa partie médiane un échangeur supplémentaire de chaleur pour évaporer la majeure partie de l'excès interne de phénol.

**4.** Procédé selon la revendication 1, caractérisé en ce que l'appareillage de distillation comporte, dans sa partie supérieure, un échangeur supplémentaire de chaleur pour refouler comme reflux la majeure partie de l'excès de phénol interne.

**5.** Procédé selon la revendication 1, caractérisé en ce que, pour séparer efficacement les produits de départ d'avec du produit de réaction volatil, on introduit un générateur supplémentaire d'azéotrope.

**6.** Procédé selon la revendication 1, caractérisé en ce qu'un acide à action catalytique que l'on introduit possède une valeur de $pK_a$ inférieure à 0.

**7.** Procédé selon la revendication 1, caractérisé en ce qu'un des acides catalytiquement actifs est de l'acide toluènesulfonique, de l'acide benzènesulfonique, de l'acide sulfurique ou de l'acide méthanesulfonique.

**8.** Procédé selon la revendication 1, caractérisé en ce que la quantité d'acide se situe entre 5 et 0,02 mol%, par rapport à un équivalent de benzyle introduit.

**9.** Procédé selon la revendication 1, caractérisé en ce qu'on conduit le procédé dans un intervalle de pression compris entre 10 mbar et 1 bar.

**10.** Procédé selon la revendication 1, caractérisé en ce que, dans la zone de la colonne correspondant à la réaction, le rapport en le constituant phénolique et la somme des produits et des équivalents benzyliques n'ayant pas réagi est en chaque endroit supérieur à 2.